(19) Europäisches Patentamt
European Patent Office
Office européen des brevets

(11) Publication number: 0 061 840
A2

# (12) EUROPEAN PATENT APPLICATION

(21) Application number: 82301029.3

(22) Date of filing: 01.03.82

(51) Int. Cl.³: C 07 D 249/08
A 01 N 43/64

(30) Priority: 30.03.81 GB 8109922

(43) Date of publication of application:
06.10.82 Bulletin 82/40

(84) Designated Contracting States:
BE CH DE FR GB IT LI NL

(71) Applicant: IMPERIAL CHEMICAL INDUSTRIES PLC
Imperial Chemical House Millbank
London SW1P 3JF(GB)

(72) Inventor: Noon, Robert Andrew
10 Dawson Road
Cricklewood London(GB)

(72) Inventor: Shephard, Margaret Claire
14 Lambourne Drive
Maidenhead Berkshire(GB)

(72) Inventor: Worthington, Paul Anthony
4 Oakhurst Road Maidenhead Court Road
Maidenhead Berkshire SL6 8H7(GB)

(72) Inventor: Gravestock, Michael Barry
10 Dawlish Avenue Cheadle Hulme
Cheadle Cheshire SK8 6JF(GB)

(74) Representative: Alner, Henry Giveen Hamilton et al,
Imperial Chemical Industries PLC Legal Department: Patents Thames House North Millbank
London SWIP 4QG(GB)

(54) **Triazole compounds, a process for preparing them, their use as plant fungicides and fungicidal compositions containing them.**

(57) Compounds of formula:

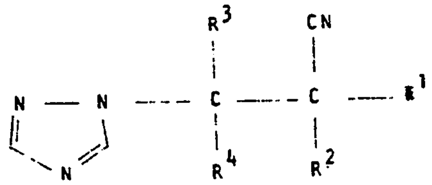

wherein $R^1$ and $R^2$ are optionally substituted phenyl and $R^3$ and $R^4$ are hydrogen, lower alkyl or optionally substituted aryl; and their isomers, acid addition salts and metal complexes compounds have fungicidal activity.

EP 0 061 840 A2

Croydon Printing Company Ltd

# TRIAZOLE COMPOUNDS, A PROCESS FOR PREPARING THEM, THEIR USE AS PLANT FUNGICIDES AND FUNGICIDAL COMPOSITIONS CONTAINING THEM

This invention relates to triazole compounds useful as fungicides, to a process for preparing them, to fungicidal compositions containing them, and to a method of combating fungal infections in plants using them.

There are disclosed in Belgian Patent No.867245 a broad class of 1,2,4-triazole derivatives having the géneral formula:

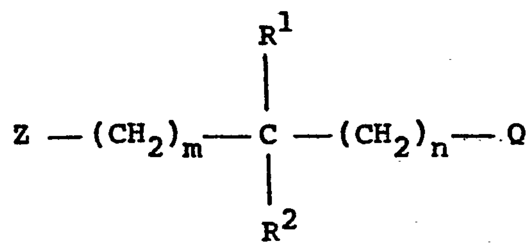

and these are stated to possess fungicidal activity. It has been found that a narrow range of unexemplified triazole compounds within this broad class have a degree of fungicidal activity far greater, indeed most surprisingly so, than the compounds illustrated in the Belgian Specification. To reach these compounds it is necessary to select specific values for Z, m, $R^1$, $R^2$, n and Q from the wide range of values specified.

The triazole compounds of the present invention have the general formula (I):

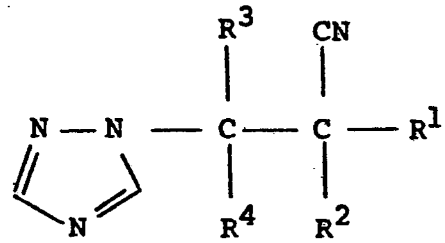

(Formula I)

wherein $R^1$ and $R^2$ are optionally substituted phenyl and $R^3$ and $R^4$ are hydrogen, lower alkyl or optionally substituted aryl; and isomers, acid addition salts and metal complexes thereof.

The compounds of the invention can contain chiral centres. Such compounds are generally obtained in the form of racemic mixtures. However, these and other mixtures can be separated into the individual isomers by methods known in the art.

The alkyl groups can be straight or branched chain groups having 1 to 6, e.g. 1 to 4, carbon atoms; examples are methyl, ethyl, propyl (n- or iso-propyl) and butyl (n-, sec-, iso- or t-butyl).

Examples of suitable substituents for the phenyl moieties are halogen (e.g. fluorine, chlorine or bromine), $C_{1-5}$ alkyl [e.g. methyl, ethyl, propyl (n- or iso-propyl) and butyl (n-, sec-, iso- or t-butyl], $C_{1-4}$ alkoxy (e.g. methoxy and ethoxy), halo- $C_{1-4}$ alkyl (e.g. trifluoromethyl or 1, 1, 2, 2-tetrafluoroethyl), halo-$C_{1-4}$ alkoxy (e.g. trifluoromethoxy or 1, 1, 2, 2-tetrafluoroethoxy), nitro, cyano, aryl (e.g. phenyl), or benzyl each optionally ring substituted (e.g. with halogen) aryloxy (eg. benzyloxy) optionally ring substituted (e.g. with halogen), alkylenedioxy, haloalkylenedioxy (e.g. difluoromethylenedioxy), amino, mono- or di- $C_{1-4}$ alkylamino (e.g. dimethylamino), and hydroxy. Suitably the phenyl moieties are unsubstituted or substituted with one, two or three ring substituents as defined above. Preferably the phenyl moieties have a single ring substituent in the 2-or 4- position. Examples of these groups are phenyl, 2-, 3-or 4-chlorophenyl, 2,4- or 2,6-dichlorophenyl, 2-, 3- or 4-fluorophenyl, 2,4- or 2,6-difluorophenyl, 2-, 3- or 4- bromophenyl, 2-chloro-4-fluorophenyl, 2-fluoro-4-chloro-phenyl, 2-chloro-6-fluorophenyl, 2-, 3- or 4-methoxyphenyl, 2, 4-dimethoxyphenyl, 2-, 3- or 4-ethoxy-phenyl, 2-, 3- or 4-nitrophenyl, 2-chloro-4-nitrophenyl, 2-chloro-5-nitrophenyl, 2-, 3- or 4-methylphenyl, 2,4-dimethylphenyl, 2-, 3- or 4-*t*-butylphenyl, 2-, 3- or 4-trifluoromethylphenyl, 2-, 3- or 4-trifluoromethoxyphenyl, 2-, 3- or 4-(1,1,2,2-tetrafluoroethyl)phenyl, 2,3-(difluoromethylenedioxy)phenyl, 2-fluoro-4-methoxyphenyl, 2-methoxy-4-fluorophenyl, 2-methoxy-4-chlorophenyl, 2-, 3- or 4-phenoxyphenyl, 2-, 3- or 4-phenylphenyl (2-, 3- or 4-biphenylyl), 2-, 3- or 4- benzylphenyl, 2-, 3- or 4-benzyloxyphenyl, 2-, 3- or 4-(4-chloro- or 4-fluorobenzyloxy)phenyl, 2-, 3- or 4-amino-phenyl, 2-, 3- or 4-(N,N-dimethylamino)phenyl, 2-, 3- or 4-hydroxyphenyl, 2-, 3- or 4-carboxyphenyl, 2-, 3- or 4-(methoxycarbonyl)phenyl.

In a further aspect, therefore, the invention provides compounds of formula II.

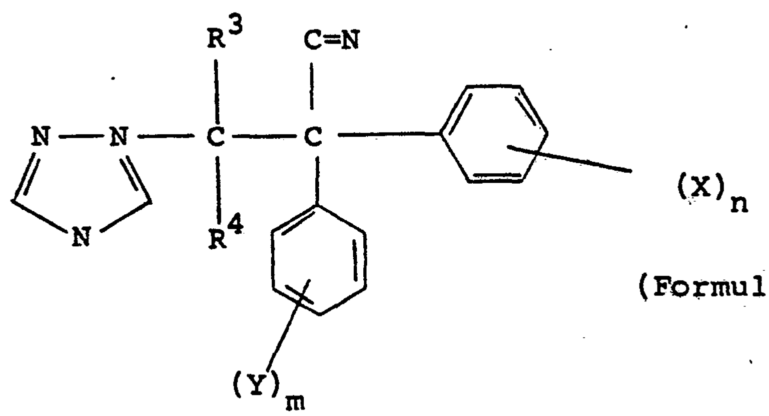

(Formula II)

wherein $R^3$ and $R^4$ are hydrogen, alkyl containing from 1 to 5 carbon atoms or optionally-substituted aryl; X and Y, which may be the same or different are halogen, cyano, nitro, amino, alkylamino especially dimethylamino, alkyl, hydroxy haloalkyl, alkoxy, haloalkoxy, aryl especially

optionally substituted phenyl, optionally substituted benzyl, aryloxy, alkylenedioxy or haloalkylenedioxy; n and m are 0, 1, 2 or 3; and isomers, acid addition salts and metal complexes thereof.

In a further aspect the invention provides compounds according to Formula II above wherein X and Y are halogen, methyl, methoxy or cyano; n and m are 1 or 2; and $R^3$ and $R^4$ are both hydrogen.

The salts with inorganic or organic acid e.g. hydrochloric, nitric, sulphuric, acetic, p-toluenesulphonic or oxalic acid.

Suitably the metal complex is one including, as the metal, copper, zinc, manganese or iron. It preferably has the general formula:

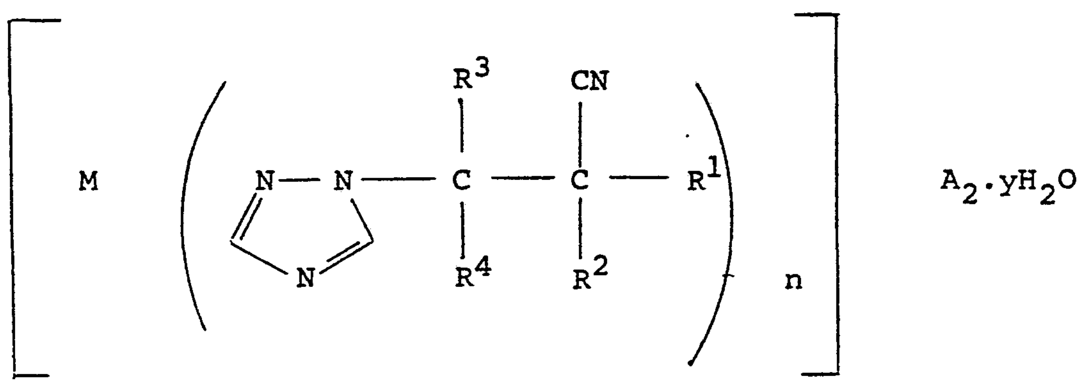

wherein $R^1$, $R^2$, $R^3$ and $R^4$ are as defined above, M is a metal, A is an anion (e.g. a chloride, bromide, iodide, nitrate, sulphate or phosphate anion), n is 2 or 4 and y is 0 or an integer of 1 to 12.

Examples of the compounds of the invention corresponding to the general formula:-

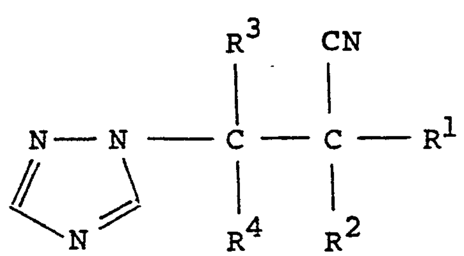

are shown in Table I below.

TABLE I

| COMPOUND | $R^1$ | $R^2$ | $R^3$ | $R^4$ | MELTING POINT (°C) |
|---|---|---|---|---|---|
| 1 | $C_6H_5$ | $C_6H_5$ | H | H | 127-128 |
| 2 | 4-Cl-$C_6H_4$ | 4-F-$C_6H_4$ | H | H | 120-121 |
| 3 | 4-F-$C_6H_4$ | 4-F-$C_6H_4$ | H | H | 115-117 |
| 4 | 4-Cl-$C_6H_4$ | 4-Cl-$C_6H_4$ | H | H | 115 |
| 5 | 4-F-$C_6H_4$ | 2-F-$C_6H_4$ | H | H | 101-102 |
| 6 | 4-F-$C_6H_4$ | 2-Cl-$C_6H_4$ | H | H | 154-155 |
| 7 | 4-Cl-$C_6H_4$ | 2-Cl-$C_6H_4$ | H | H | |
| 8 | 2-4-di-Cl$C_6H_3$ | $C_6H_5$ | H | H | 153-154 |
| 9 | 2-Cl-4-$CH_3$-$C_6H_3$ | 4-F-$C_6H_4$ | H | H | |
| 10 | 2-F-4-$CH_3$-$C_6H_3$ | 4-F-$C_6H_4$ | H | H | |
| 11 | 2-Cl-4-$OCH_3$-$C_6H_3$ | 4-F-$C_6H_4$ | H | H | |
| 12 | 2-F-4-$OCH_3$-$C_6H_3$ | 4-F-$C_6H_4$ | H | H | |
| 13 | 2-Cl-4-CN-$C_6H_3$ | 4-F-$C_6H_4$ | H | H | |
| 14 | 2-F-4-CN-$C_6H_3$ | 4-F-$C_6H_4$ | H | H | |

The compounds of general formula (III)

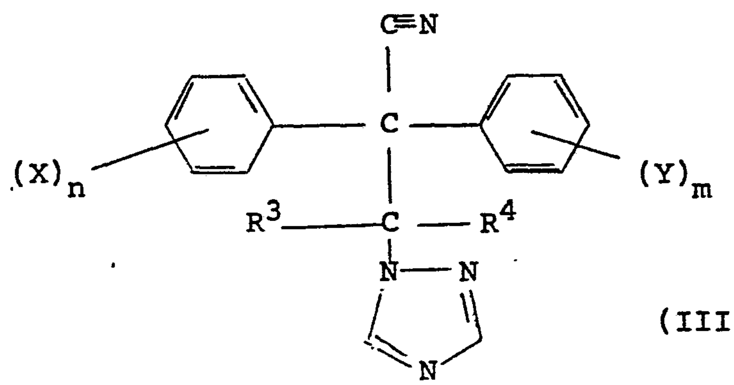

(III)

in which $R^3$, $R^4$, X, Y, n and m are as defined above may be produced by reacting a compound of general formula (IV)

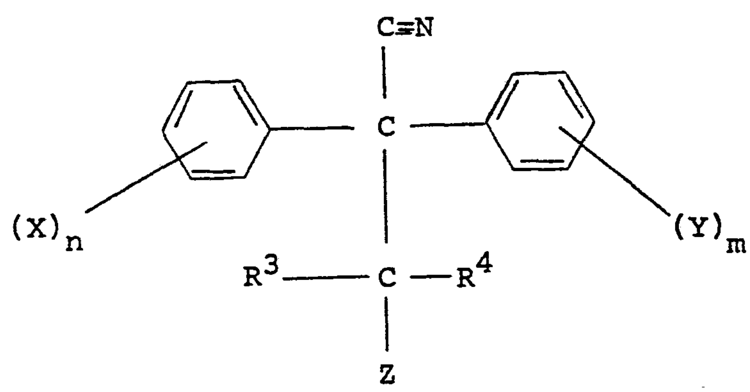

(IV)

wherein $R^3$, $R^4$, X, Y, n and m are as defined above and Z is a leaving group (for example chlorine, bromine, mesylate or tosylate), with 1,2,4-triazole either in the presence of an acid-binding agent or in the form of one of its alkali metal salts in a convenient solvent. Suitably the compound of general formula (IV) is reacted at 20-100° with the sodium salt of 1,2,4-triazole (the salt can be prepared by adding either sodium hydride or sodium methoxide to 1,2,4-triazole) in a convenient solvent such as

acetonitrile, methanol, ethanol or dimethylformamide. The product can be isolated by pouring the reaction mixture into water and recrystallising the solid formed from a convenient solvent.

The compounds of general (IV) can be prepared by reacting an alcohol (V)

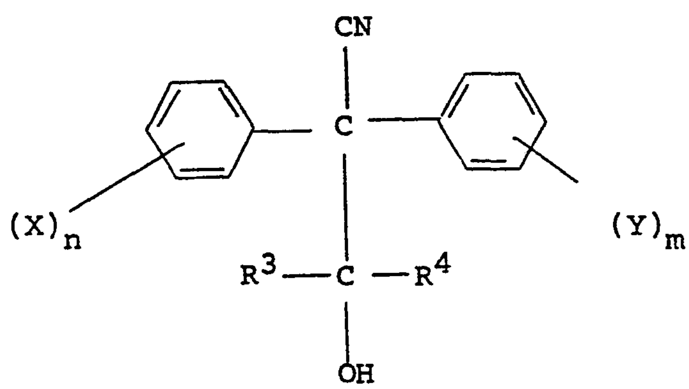

(V)

with phosphorus trichloride, phosphorus pentachloride or thionyl chloride in a convenient solvent such as toluene to give the chloride. The alcohol can be converted to the bromide using phosphorus tribromide, the mesylate using methane sulphonyl chloride in pyridine, or the tosylate using *p*-toluenesulphonyl chloride in pyridine.

The alcohols of general formula (V) can be prepared by hydroxyalkylating the substituted acetonitrile (VI) with an aldehyde or ketone (VII)

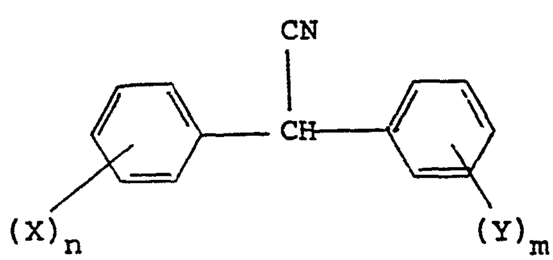

(VI)

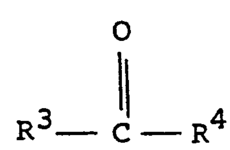

(VII)

in a solvent such as pyridine, dimethylformamide, or dimethyl sulphoxide with a basic catalyst such as sodium hydroxide, sodium ethoxide, or a quaternary ammonium hydroxide. The nitriles of general formula (VI) are prepared by reacting the compounds (VIII)

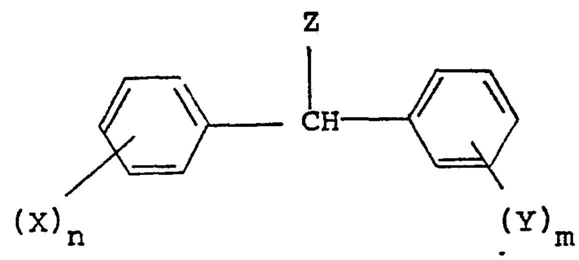

(VIII)

with a metal cyanide in a solvent such as dimethyl formamide or dimethyl sulphoxide at temperatures of 60-200°C.

Compounds of general formula (VI) may also be prepared by reacting an aldehyde (IX) with a metal cyanide in the presence of a substituted benzene (X).

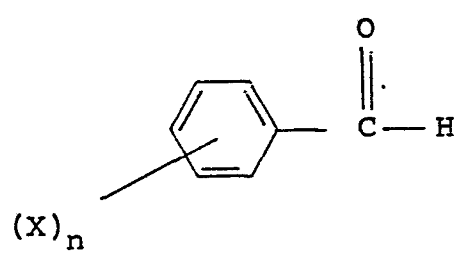

(IX)

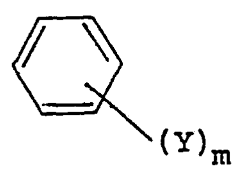

(X)

The compounds of general formula (VIII) are prepared by treating the corresponding alcohols (XI)

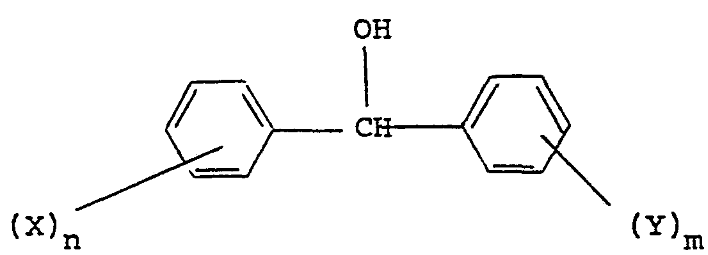

(XI)

with the normal reagents as previously indicated.

The alcohols of general formula (XI) are prepared by reacting the aldehyde (IX) with an aryl magnesium halide in a convenient solvent such as ether or tetrahydrofuran. They may also be prepared by reacting the benzophenone (XII) with a reducing agent such as a dissolving metal hydride e.g. $NaBH_4$ or $LiAlH_4$

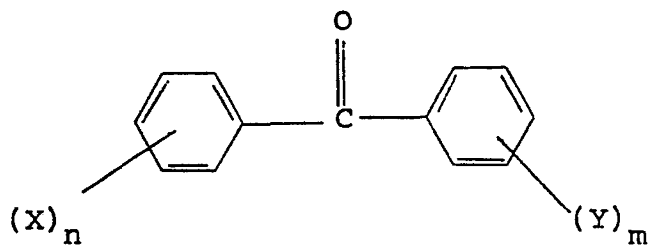

(XII)

Compounds of general formula (IV) may also be prepared by reacting (VI) with a compound (XIII)

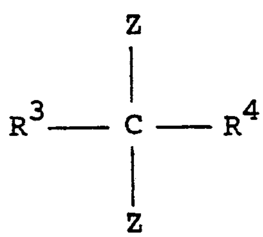

(XIII)

in a convenient solvent such as dimethyl formamide or dimethyl sulphoxide using a base such as sodium hydroxide or sodium methoxide.

This invention further includes within its scope the novel intermediates having the structural formulae below

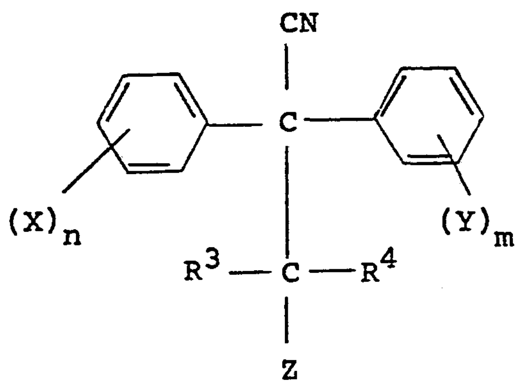

(IV)

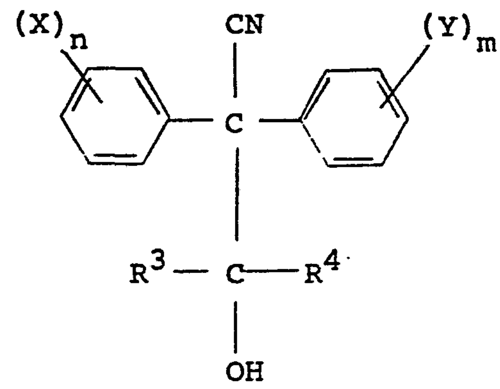

(V)

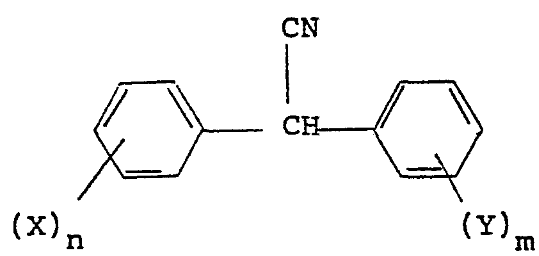

(VI)

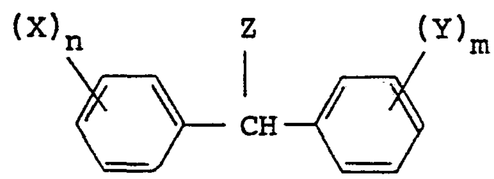

(VIII)

wherein X and Y and n and m are as defined (for the phenyl ring substituents) above; and Z is a leaving group, such as, for example, $-OSO_2CH_3$; provided that when X is H then Y is not H or 2,4-dichloro, and that when X is 4-chloro, Y is not 2-chloro or 4-chloro.

In particular the invention provides, as novel intermediates, compounds of the formula above wherein n and m are 1; Z is as defined; and X and Y are chloro- or fluoro-, preferably at least one of X and Y being fluoro; particularly preferred intermediates have one fluorine substituent at the 2- or 4- position of one phenyl ring with a fluorine or chlorine substituent at the 2- or 4-position of the other phenyl ring.

The salts and metal complexes of the compounds of general formula (I) can be prepared from the latter in known manner. For example, the complexes can be made by reacting the uncomplexed compound with a metal salt in a suitable solvent.

The compounds, salts and metal complexes are active fungicides, particularly against the diseases:-

Piricularia oryzae on rice
Puccinia recondita, Puccinia striiformis and other rusts on wheat, Puccinia hordei, Puccinia striiformis and other rusts on barley, and rusts on other hosts e.g. coffee, apples, vegetables and ornamental plants
Erysiphe graminis (powdery mildew) on barley and wheat and other powdery mildews on various hosts such as Sphaerotheca fuliginea on cucurbits (e.g. cucumber), Podosphaera leucotricha on apples and Uncinula necator on vines
Helminthosporium spp. and Rhynchosporium spp. on cereals
Cercospora arachidicola on peanuts and other Cercospora species on for example sugar beet, bananas and soya beans
Botrytis cinerea (grey mould) on tomatoes, strawberries, vines and other hosts
Venturia inaequalis (scab) on apples

Some of the compounds have also shown a broad range of activities against fungi in vitro. They have activity against various post-harvest diseases on fruit (e.g. Penicillium digatatum and italicum on oranges and Gloeosporium musarum on bananas). Further some of the compounds are active as seed dressings against:

Fusarium spp., Septoria spp., Tilletia spp. (i.e. bunt, a seed borne disease of wheat), Ustilago spp., Helminthosporium spp. on cereals, Rhizoctonia solani on cotton and Corticium sasakii on rice.

The compounds can move acropetally in the plant tissue. Moreover, the compounds can be volatile enough to be active in the vapour phase against fungi on the plant.

The compounds are also useful for the treatment of candidiasis and human dermatophyte infections.

The compounds may be used as such for fungicidal purposes but are more conveniently formulated into compositions for such usage. The invention thus provides also a fungicidal composition comprising a compound of general formula (I) or a salt or complex thereof as hereinbefore defined, and a carrier or diluent.

The invention also provides a method of combating fungal diseases in a plant, which method comprises applying to the plant, to seed of the plant or to the locus of the plant or seed a compound or a salt or complex thereof as hereinbefore defined.

The compounds, salts and complexes can be applied in a number of ways, for example they can be formulated or unformulated, directly to the foliage of a plant, to seeds or to other medium in which plants are growing or are to be planted, or they can be sprayed on, dusted on or applied as a cream or paste formulation, or they can be applied as a vapour. Application can be to any part of the plant, bush or tree, for example to the foliage, stems, branches or roots, or to soil surrounding the roots, or to the seed before it is planted.

The term "plant" as used herein includes seedlings, bushes and trees. Furthermore, the fungicidal method of the invention includes preventative, protectant, prophylactic and eradicant treatment.

The compounds are preferably used for agricultural and horticultural purposes in the form of a composition. The type of composition used in any instance will depend upon the particular purpose envisaged.

The compositions may be in the form of dusting powders or granules comprising the active ingredient and a solid diluent or carrier, for example fillers such as kaolin, bentonite, kieselguhr, dolomite, calcium carbonate, talc, powdered magnesia, Fuller's earth, gypsum, Hewitt's earth, diatomaceous earth and China clay. Such granules can be preformed granules suitable for application to the soil without further treatment. These granules can be made either by impregnating pellets of filler with the active ingredient or by pelleting a mixture of the active ingredient and powdered filler. Compositions for dressing seed, for example, may comprise an agent (for example a mineral oil) for assisting the adhesion of the composition to the seed; alternatively the active ingredient can be formulated for seed dressing purposes using an organic solvent (for example N-methylpyrrolidone or dimethylformamide).

The compositions may also be in the form of dispersible powders, granules or grains comprising a wetting agent to facilitate the dispersion in liquids of the powder or grains which may contain also fillers and suspending agents.

The aqueous dispersions or emulsions may be prepared by dissolving the active ingredient(s) in an organic solvent optionally containing wetting, dispersing or emulsifying agent(s) and then adding the mixture to water which may also contain wetting, dispersing or emulsifying agent(s). Suitable organic solvents are ethylene dichloride, isopropyl alcohol, propylene glycol, diacetone alcohol, toluene, kerosene, methylnaphthalene, the xylenes, trichloroethylene, furfuryl alcohol, tetrahydrofurfuryl alcohol, and glycol ethers (e.g. 2-ethoxyethanol and 2-butoxyethanol).

The compositions to be used as sprays may also be in the form of aerosols wherein the formulation is held in a container under pressure in the presence of a propellant, e.g. fluorotrichloromethane or dichlorodifluoromethane.

The compounds can be mixed in the dry state with a pyrotechnic mixture to form a composition suitable for generating in enclosed spaces a smoke containing the compounds.

Alternatively, the compounds may be used in a microencapsulated form.

By including suitable additives, for example additives for improving the distribution, adhesive power and resistance to rain on treated surfaces, the different compositions can be better adapted for various utilities.

The compounds can be used as mixtures with fertilisers (e.g. nitrogen-, potassium- or phosphorus-containing fertilisers). Compositions comprising only granules of fertiliser incorporating, for example coated with, the compound, are preferred. Such granules suitably contain up to 25% by weight of the compound. The invention therefore also provides a fertiliser composition comprising the compound of general formula (I) or a salt or metal complex thereof.

The compositions may also be in the form of liquid preparations for use as dips or sprays which are generally aqueous dispersions or emulsions containing the active ingredient in the presence of one or more surfactants e.g. wetting agent(s), dispersing agent(s), emulsifying agent(s) or suspending agent(s). These agents can be cationic, anionic or non-ionic agents. Suitable cationic agents are quaternary ammonium compounds, for example cetyltrimethylammonium bromide.

Suitable anionic agents are soaps, salts of aliphatic monoesters of sulphuric acid (for example sodium lauryl sulphate), and salts of sulphonated aromatic compounds (for

example sodium dodecylbenzenesulphonate, sodium, calcium or ammonium lignosulphonate, butylnaphthalene sulphonate, and a mixture of sodium diisopropyl- and triisopropylnaphthalene sulphonates).

Suitable non-ionic agents are the condensation products of ethylene oxide with fatty alcohols such as oleyl or cetyl alcohol, or with alkyl phenols such as octyl- or nonyl-phenol and octylcresol. Other non-ionic agents are the partial esters derived from long chain fatty acids and hexitol anhydrides, the condensation products of the said partial esters with ethylene oxide, and the lecithins. Suitable suspending agents are hydrophilic colloids (for example polyvinylpyrrolidone and sodium carboxymethylcellulose), and the vegetable gums (for example gum acacia and gum tragacanth).

The compositions for use as aqueous dispersions or emulsions are generally supplied in the form of a concentrate containing a high proportion of the active ingredient(s), the concentrate to be diluted with water before use. These concentrates often should be able to withstand storage for prolonged periods and after such storage be capable of dilution with water in order to form aqueous preparations which remain homogeneous for a sufficient time to enable them to be applied by conventional spray equipment. The concentrates may conveniently contain up to 95%, suitably 10-85%, for example 25-60%, by weight of the active ingredient(s). These concentrates suitably contain organic acids (e.g. alkaryl or aryl sulphonic acids such as xylenesulphonic acid or dodecylbenzenesulphonic acid) since the presence of such acids can increase the solubility of the active ingredient(s) in the polar solvents often used in the concentrates. The concentrates suitably contain also a high proportion of surfactants so that sufficiently stable emulsions in water can be obtained. After dilution to form aqueous preparations, such preparations may contain varying

amounts of the active ingredient(s) depending upon the intended purpose, but an aqueous preparation containing 0.0005% or 0.01% to 10% by weight of active ingredient(s) may be used.

The compositions of this invention can comprise also other compound(s) having biological activity, e.g. compounds having similar or complementary fungicidal activity or compounds having plant growth regulating, herbicidal or insecticidal activity.

The other fungicidal compound can be for example one which is capable of combating ear diseases of cereals (e.g. wheat) such as Septoria, Giberella and Helminthosporium spp., seed and soil borne diseases and downy and powdery mildews on grapes and powdery mildew and scab on apple etc. These mixtures of fungicides can have a broader spectrum of activity than the compound of general formula (I) alone; further the other fungicide can have a synergistic effect on the fungicidal activity of the compound of general formula (I). Examples of the other fungicidal compound are imazalil, benomyl, carbendazim, thiophanate-methyl, captafol, captan, sulphur, triforine, dodemorph, tridemorph, pyrazophos, furalaxyl, ethirimol, dimethirimol, bupirimate, chlorothalonil, vinclozolin, procymidone, iprodione, metalaxyl, forsetyl-aluminium, carboxin, oxycarboxin, fenarimol, nuarimol, fenfuram, methfuroxan, nitrotal-isopropyl, triadimefon, thiabendazole, etridiazole, triadimenol, biloxazol, dithianon, binapacryl, quinomethionate, guazitine, dodine, fentin acetate, fentin hydroxide, dinocap, folpet, dichlofluanid, ditalimphos, kitazin, cycloheximide, dichlobutrazol, a dithiocarbamate, a copper compound, a mercury compound, 1-(2-cyano-2-methoxyiminoacetyl)-3-ethyl urea, fenapanil, ofurace, propiconazole, etaconazole and fenpropemorph.

The compounds of general formula (I) can be mixed with soil, peat or other rooting media for the protection of plants against seed-borne, soil-borne or foliar fungal diseases.

Suitable insecticides are pirimor, croneton, dimethoate, metasystox and formothion.

Examples of suitable plant growth regulating compounds are the gibberellins (e.g. $GA_3$, $GA_4$ or $GA_7$), the auxins (e.g. indoleacetic acid, indole-butyric acid, naphthoxyacetic acid or naphthylacetic acid), the cytokinins (e.g. kinetin, diphenylurea, benzimidazole, benzyladenine or BAP), phenoxyacetic acids (e.g. 2,4-D or MCPA), substituted benzoic acids (e.g. triiodobenzoic acid), morphactins (e.g. chlorfluorecol), maleic hydrazide, glyphosate, glyphosine, long chain fatty alcohols and acids, dikegulac, fluoridamid, mefluidide, substituted quaternary ammonium and phosphonium compounds (e.g. chlormequat or chlorphonium), ethepon, carbetamide, methyl-3,6-dichloranisate, daminozide, asulam, abscissic acid, isopyrimol, 1(4-chlorophenyl)-4,6-dimethyl-2-oxo-1,2-dihydropyridine-3-carboxylic acid, hydroxybenzonitriles (e.g. bromoxynil), difenzoquat, benzoylpropethyl 3,6-dichloropicolinic acid.

The following Example illustrates the invention; the temperatures are given in degrees Centigrade (°C).

EXAMPLE 1

This Example illustrates the preparation of the compound.

1-(1,2,4-Triazol-1-yl)-2-cyano-2-(4-chlorophenyl)-2-(4-fluorophenyl)ethane. (Compound No 2 of Table 1)

Stage 1: 4-Chloro-4'-fluorobenzophenone (0.1 mol) was dissolved in methanol (100 ml) and cooled to 0°. $NaBH_4$ (0.1 mol) was added portionwise and stirred at 0° for 1 hour and at room temperature for 1 hour. The methanol was removed *in vacuo* and the residue dissolved in ether (200 ml), washed with water (3 x 150 ml), and dried over anhydrous sodium sulphate. Removal of the solvent gave 4-chloro-4'-fluorobenzhydrol (95%) as an oil which slowly solidified.

Stage 2: Phosphorus tribromide (0.025 mol) was added dropwise to the 4-chloro-4'-fluorobenzhydrol (0.05 mol) at 0°. The solution was stirred at 0° for 1 hour and at room temperature for 2 hours. After pouring into ice water (200 ml) the solution was extracted with ether (150 ml), washed with water (2 x 100 ml) and dried over anhydrous sodium sulphate. Removal of the solvent gave an oil which distilled at reduced pressure to give 4-chloro-4'-fluorodiphenyl bromomethane (75%) as a pale yellow liquid b.p. 118-119°/0.1 mm Hg.

Stage 3: 4-Chloro-4'-fluorodiphenyl bromomethane (0.04 mol) was added at 125° to anhydrous cuprous cyanide (0.044 mol). An exothermic reaction took place and the temperature rose to 180°. The reaction was heated at 125° for 1 hour. Chloroform (100 ml) was added and the solution filtered. The filtrate was evaporated in vacuo and distilled at reduced pressure to give 4-chloro-4'-fluorodiphenyl acetonitrile (80%) as a colourless oil b.p. 129-130°/0.1 mm Hg.

Stage 4: 4-Chloro-4'-fluorodiphenyl acetonitrile (0.024 mol) and paraformaldehyde (0.027 mol) were stirred in dimethyl sulphoxide (20 ml) at room temperature, containing 0.5 N sodium ethoxide (0.5 ml) for two hours. The solution was poured into water (50 ml) acidified with glacial acetic acid. After extracting with ether (150 ml) the solution was washed with water (2 x 100 ml) and dried over anhydrous sodium sulphate. Removal of the solvent gave 2-cyano-2-(4-chlorophenyl)-2-(4-fluorophenyl)ethanol (90%) as a colourless oil.

Stage 5: Mesyl chloride (0.03 mol) was added dropwise to a solution of 2-cyano-2-(4-chlorophenyl)-2-(4-fluorophenyl) ethanol (0.015 mol) in pyridine (40 ml) at 0°. After stirring at room temperature overnight the solution was poured into water, and the solid filtered off. Recrystallisation from 60-80 petroleum ether/$CHCl_3$ gave

2-cyano-2-(4-chlorophenyl)-2-(4-fluorophenyl)-ethyl methane sulphonate (90%) m.p. 136-137°.

Stage 6: Sodium hydride (0.011 mol) was suspended in DMF (20 ml) and 1,2,4-triazole (0.011 mol) was added portionwise, then stirred at room temperature until the effervescence ceased. 2-cyano-2-(4-chlorophenyl)-2-(4-fluorophenyl)-ethyl methane sulphonate (0.0056 mol) was added portionwise at room temperature and the mixture heated at 120° for 5 hours. The reaction was poured into water to give a solid which recrystallised from 60-80 petroleum ether/$CHCl_3$ to give the title compound (50%) as a white crystalline solid m.p. 120-121°.

EXAMPLE 2

The compounds were tested against a variety of foliar fungal diseases of plants. The technique employed was as follows.

The plants were grown in John Innes Potting Compost (No 1 or 2) in 4 cm diameter minipots. A layer of fine sand was placed at the bottom of the pots containing the dicotyledonous plants to facilitate uptake of test compound by the roots. The test compounds were formulated either by bead milling with aqueous Dispersol T or as a solution in acetone or acetone/ethanol which was diluted to the required concentration immediately before use. For the foliage diseases, suspensions (100 ppm active ingredient) were sprayed on to the soil. Exceptions to this were the tests on Botrytis cinerea, Plasmopara viticola and Venturia inaequalis. The sprays were applied to maximum retention and the root drenches to a final concentration equivalent to approximately 40 ppm a.i./dry soil. Tween 20, to give a final concentration of 0.05%, was added when the sprays were applied to cereals.

For most of the tests the compound was applied to the soil (roots) and to the foliage (by spraying) one or two days before the plant was inoculated with the diseases. An exception was the test on Erysiphe graminis in which the plants were inoculated 24 hours before treatment. After inoculation, the plants were put into an appropriate environment to allow infection to take place and then incubated until the disease was ready for assessment. The period between inoculation and assessment varied from four to fourteen days according to the disease and environment. The disease control was recorded by the following grading:-

4 = no disease
3 = trace - 5% of disease on untreated plants
2 = 6-25% of disease on untreated plants
1 = 26-59% of disease on untreated plants
0 = 60-100% of disease on untreated plants

The results are shown in Table II.

TABLE II

| COMPOUND NUMBER | PUCCINIA RECONDITA (WHEAT) | ERYSIPHE GRAMINIS (BARLEY) | PIRICULARIA ORYZAE (RICE) | PLASMOPARA VITICOLA (VINE) | PHYTOPHTHORA INFESTANS (TOMATO) | BOTRYTIS CINEREA (GRAPE) | CERCOSPORA ARACHIDICOLA (PEANUT) | VENTURIA INAEQUALIS (APPLE) |
|---|---|---|---|---|---|---|---|---|
| 1 | 4 | 4 | 4 | 0 | 0 | 1 | 4 | 4 |
| 2 | 4 | 4 | 4 | 0 | 0 | 3 | 4 | 4 |
| 3 | 4 | 4 | 4 | 0 | 0 | 2 | 4 | 4 |
| 4 | - | - | - | - | - | - | - | - |
| 5 | 4 | 4 | - | 1 | - | - | 3 | 4 |
| 6 | 4 | 4 | - | 0 | - | - | 0 | 2 |
| 7 | 4 | 4 | - | 2 | - | 0 | 0 | 0 |
| 8 | 4 | 4 | - | 2 | - | 0 | 0 | 0 |

"-" means not tested.

EXAMPLE 3

An emulsifiable concentrate was made up by mixing the ingredients, and stirring the mixture until all the constituents were dissolved.

| | |
|---|---|
| Compound No.1 of Table I | 10% |
| Ethylene dichloride | 40% |
| Calcium dodecylbenzenesulphate | 5% |
| "Lubrol" L | 10% |
| "Aromasol" H | 35% |

EXAMPLE 4

A composition in the form of grains readily dispersible in a liquid, e.g. water, was prepared by grinding together the first three ingredients in the presence of added water and then mixing in the sodium acetate. The resultant mixture was dried and passed through a British Standard mesh sieve, size 44-100, to obtain the desired size of grains.

| | |
|---|---|
| Compound No.2 of Table I | 50% |
| "Dispersol" T | 25% |
| "Lubrol" APN5 | 1.5% |
| Sodium acetate | 23.5% |

EXAMPLE 5

The ingredients were all ground together to produce a powder formulation readily dispersible in liquids.

| | |
|---|---|
| Compound No.3 of Table I | 45% |
| "Dispersol" T | 5% |
| "Lissapol" NX | 0.5% |
| "Cellofas" B600 | 2% |
| Sodium acetate | 47.5% |

EXAMPLE 6

The active ingredient was dissolved in a solvent and the resultant liquid was sprayed on to the granules of China clay. The solvent was then allowed to evaporate to produce a granular composition.

| | |
|---|---|
| Compound No.1 of Table I | 5% |
| China clay granules | 95% |

EXAMPLE 7

A composition suitable for use as a seed dressing was prepared by mixing the three ingredients.

| | |
|---|---|
| Compound No.2 of Table I | 50% |
| Mineral oil | 2% |
| China clay | 48% |

EXAMPLE 8

A dusting powder was prepared by mixing the active ingredient with talc.

| | |
|---|---|
| Compound No.3 of Table I | 5% |
| Talc | 95% |

EXAMPLE 9

A Col formulation was prepared by ball-milling the constituents set out below and then forming an aqueous suspension of the ground mixture with water.

| | |
|---|---|
| Compound No.4 of Table I | 40% |
| "Dispersol" T | 10% |
| "Lubrol" APN5 | 1% |
| Water | |

EXAMPLE 10

A dispersible powder formulation was made by mixing together the ingredients set out below and then grinding the mixture until all were thoroughly mixed.

| | |
|---|---|
| Compound No.1 of Table I | 25% |
| "Aerosol" OT/B | 2% |
| "Dispersol" A.C. | 5% |
| China clay | 28% |
| Silica | 40% |

EXAMPLE 11

This Example illustrates the preparation of a dispersible powder formulation. The ingredients were mixed and the mixture then ground in a comminution mill.

| | |
|---|---|
| Compound No.2 of Table I | 25% |
| "Perminal" BX | 1% |
| "Dispersol" T | 5% |
| Polyvinylpyrrolidone | 10% |
| Silica | 25% |
| China clay | 34% |

EXAMPLE 12

The ingredients set out below were formulated into a dispersible powder by mixing then grinding the ingredients.

| | |
|---|---|
| Compound No.3 of Table I | 25% |
| "Aerosol" OT/B | 2% |
| "Dispersol" A | 5% |
| China clay | 68% |

In Examples 3 to 12 the proportions of the ingredients given are by weight.

There now follows an explanation of the compositions or substances represented by the various Trade Marks and Trade Names mentioned above.

| | |
|---|---|
| LUBROL L : | a condensate of nonyl phenol 1 mole) with ethylene oxide (13 moles) |
| AROMASOL H : | a solvent mixture of alkylbenzenes |
| DISPERSOL T & AC : | a mixture of sodium sulphate and a condensate of formaldehyde with sodium naphthalene sulphonate |
| LUBROL APN5 : | a condensate of nonyl phenol (1 mole) with naphthalene oxide (5.5 moles) |
| CELLOFAS B600 : | a sodium carboxymethyl cellulose thickener |
| LISSAPOL NX : | a condensate of nonyl phenol (1 mole) with ethylene oxide (8 moles) |
| AEROSOL OT/B : | dioctyl sodium sulphosuccinate |
| PERMINAL BX : | a sodium alkyl naphthalene sulphonate |

HGHA/SPEC223/jlw
22.2.82

1. Triazole compounds of the general formula (I):

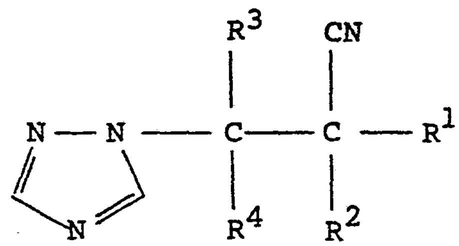

(I)

wherein $R^1$ and $R^2$ are optionally substituted phenyl and $R^3$ and $R^4$ are hydrogen, lower alkyl or optionally substituted aryl; and isomers, acid addition salts and metal complexes thereof.

2. Compounds of formula

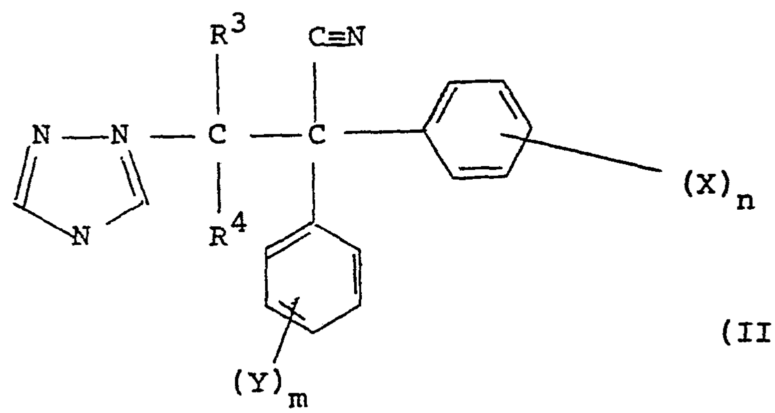

(II)

wherein $R^3$ and $R^4$ are hydrogen, alkyl containing from 1 to 5 carbon atoms or optionally-substituted aryl; X and Y, which may be the same or different are halogen, cyano, nitro, amino, alkylamino especially dimethylamino, alkyl, hydroxy, haloalkyl, alkoxy, haloalkoxy, aryl especially optionally substituted phenyl, optionally substituted benzyl, aryloxy, alkylenedioxy or haloalkylenedioxy; n and m are 0, 1, 2 or 3; and isomers, acid addition salts and metal complexes thereof.

3. Compounds according to claim 2 wherein X and Y are halogen, methyl, methoxy or cyano; n and m are 1 or 2, and $R^3$ and $R^4$ are both hydrogen.

4. A process for preparing the compounds of general formula (I) as claimed in claims 1 to 3 which comprises reacting a compound of general formula (IV)

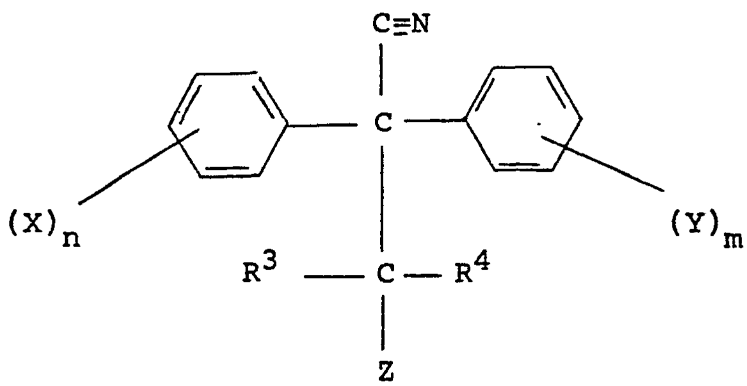

(IV)

wherein $R^3$, $R^4$, X, Y, n and m are as defined above and Z is a leaving group (for example chlorine, bromine, mesylate or tosylate), with 1,2,4-triazole either in the presence of an acid-binding agent or in the form of one of its alkali metal salts in a convenient solvent.

5. A process as claimed in claim 4 wherein the compound of general formula (IV) is reacted at 20-100° with the sodium salt of 1,2,4-triazole (the salt can be prepared by adding either sodium hydride or sodium methoxide to 1,2,4-triazole) in a convenient solvent such as acetonitrile, methanol, ethanol or dimethylformamide and thereafter, if desired isolating the product by pouring the reaction mixture into water and recrystallising the solid formed from a convenient solvent.

6. A process as claimed in claim 4 or claim 5 which comprises the preparation of compounds of general formula (IV) wherein Z is chlorine by reacting an alcohol (V)

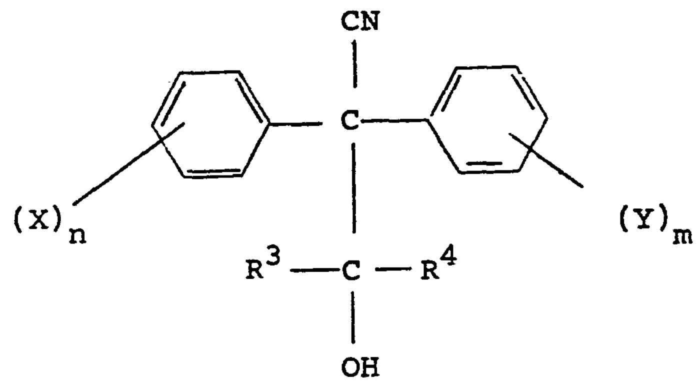

(V)

with phosphorus trichloride, phosphorus pentachloride or thionyl chloride in a convenient solvent such as toluene; or converting the alcohol to the bromide using phosphorus tribromide, or to the mesylate using methane sulphonyl chloride in pyridine, or to the tosylate using p-toluenesulphonyl chloride in pyridine.

7. A process according to claim 6 wherein the alcohols of general formula (V) are prepared by hydroxyalkylating the substituted acetonitrile (VI) with an aldehyde or ketone (VII)

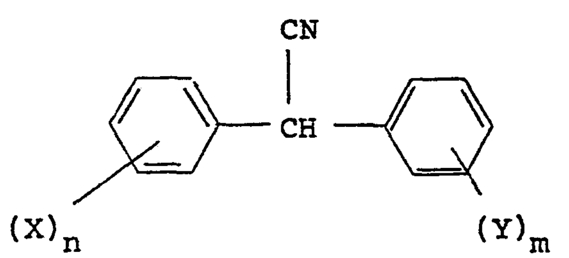

(VI)

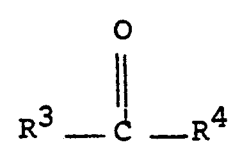

(VII)

in a solvent such as pyridine, dimethylformamide, or dimethyl sulphoxide with a basic catalyst such as sodium hydroxide, sodium ethoxide, or a quaternary ammonium hydroxide.

8. A process as claimed in claim 7 wherein the nitriles of general formula (VI) are prepared by reacting the compounds (VIII)

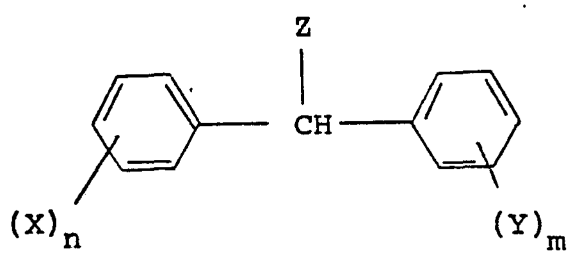

(VIII)

with a metal cyanide in a solvent such as dimethyl formamide or dimethyl sulphoxide at temperatures of from 60-200°C.

9. A process as claimed in claim 7 wherein the compounds of general formula (VI) are prepared by reacting an aldehyde (IX) with a metal cyanide in the presence of a substituted benzene (X).

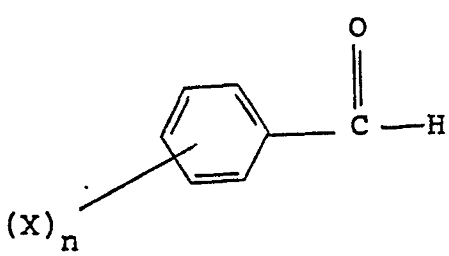

(IX)

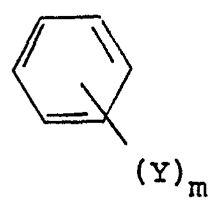

(X)

 

10. A process as claimed in claimed 8 wherein the compounds of general formula (VIII) are prepared by treating the corresponding alcohols (XI)

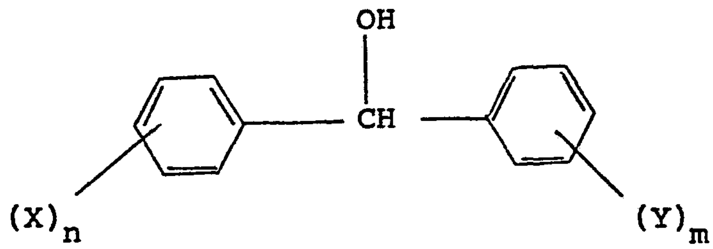

(XI)

with the normal reagents.

11. A process as claimed in claim 8 wherein the alcohols of general formula (XI) are prepared by reacting the aldehyde (IX) defined in claim 9 with an aryl magnesium halide in a convenient solvent such as ether or tetrahydrofuran or by reacting the benzophenone (XII) with a reducing agent such as a dissolving metal hydride e.g. $NaBH_4$ or $LiAlH_4$

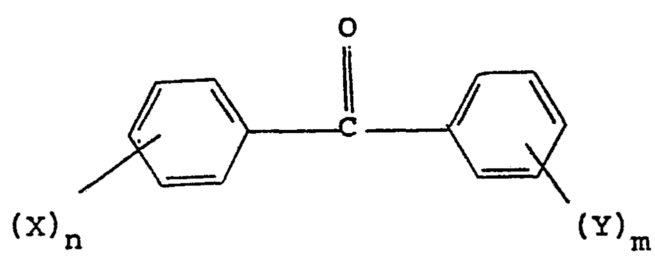

(XII)

12. A process as claimed in claim 4 wherein the compounds of general formula (II) are prepared by reacting a substituted acetonitrile (VI) as defined in claim 7 with a compound (XIII)

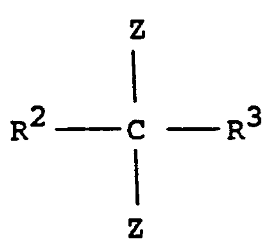

(XIII)

in a convenient solvent such as dimethylformamide or dimethyl sulphoxide using a base such as sodium hydroxide or sodium methoxide.

13. The novel intermediates having the structural formulae below

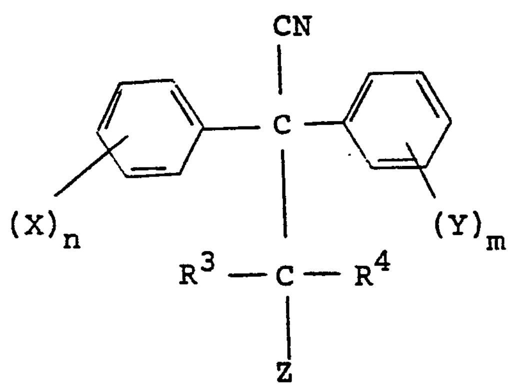

(IV)

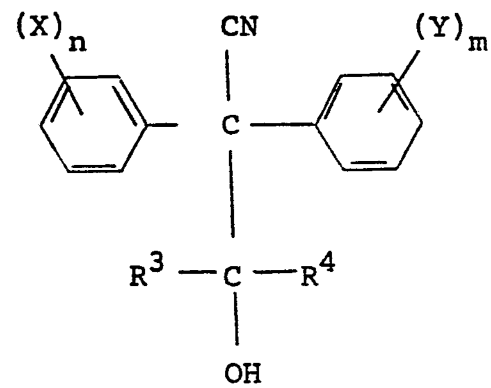

(V)

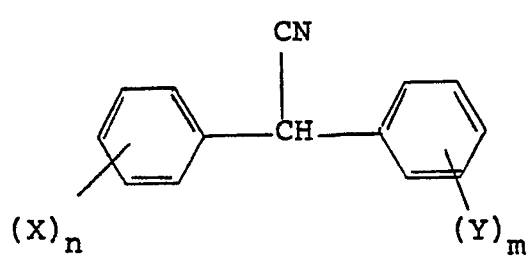

(VI)

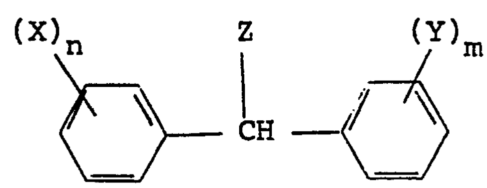

(VIII)

wherein X and Y and n and m are as defined (for the phenyl ring substituents) above; and Z is a leaving group, such as, for example, $-OSO_2CH_3$; provided that when X is H then Y is not H or 2,4-dichloro, and that when X is 4-chloro, Y is not 2-chloro or 4-chloro.

14. As novel intermediates, compounds of the formulae set out in claim 13 wherein n and m are 1; Z is as defined; and X and Y are chloro- or fluoro-, preferably at least one of X and Y being fluoro and especially those intermediates having one fluorine substituent at the 2- or 4-position of one phenyl ring and a fluorine or chlorine substituent at the 2- or 4- position of the other phenyl ring.

15. A fungicidal composition comprising, as an active ingredient, a compound, or a salt or metal complex thereof, as defined in any of claims 1 to 3 together with a carrier or diluent thereof.

16. The invention also provides a method of combating fungal diseases in a plant which comprises applying to the plant, to seed of the plant, or to the locus of the plant or seed, a compound, or a salt or metal complex thereof, as defined in any of claims 1 to 3 or a composition as defined in claim 15.